# EUROPÄISCHE PATENTSCHRIFT

(11) **EP 1 056 779 B1**
(45) Veröffentlichungstag und Bekanntmachung des Hinweises auf die Patenterteilung: **26.04.2006**
(21) Anmeldenummer: 99904614.7
(22) Anmeldetag: 25.02.1999
(51) Int. Cl.: C07K 14/755, A61K 38/37

(54) **VERFAHREN ZUR HERSTELLUNG VON FAKTOR VIII/VWF-KOMPLEX**
METHOD FOR PRODUCING A FACTOR VIII/VON WILLEBRAND FACTOR COMPLEX
PROCEDE DE PRODUCTION D'UN COMPLEXE FACTEUR VIII/FACTEUR DE VON WILLEBRAND

(30) Priorität: 27.02.1998 WO PCT/AT98/00043; 20.05.1998 AT 86698
(43) Veröffentlichungstag der Anmeldung: 06.12.2000
(73) Patentinhaber: Baxter Aktiengesellschaft, 1221 Wien (AT)
(72) Erfinder: LINNAU, Yendra, A-1220 Wien (AT); SCHÖNHOFER, Wolfgang, A-3100 St. Pölten (AT)
(74) Vertreter: Alge, Daniel
(86) Internationale Anmeldenummer: PCT/AT1999/000048
(87) Internationale Veröffentlichungsnummer: WO 1999/043712

(56) Entgegenhaltungen:
- EP-A- 0 295 645
- EP-A- 0 600 480
- WO-A-91/13093
- WO-A-93/15199
- WO-A-97/34930
- WO-A-97/39033
- WO-A-98/38219

## Beschreibung

Die Erfindung betrifft ein Verfahren zur Herstellung einer Präparation enthaltend Faktor VIII:C/vWF-Komplex.

Zur Therapie von Hämophilie-Erkrankungen werden Faktor VIII-Präparate seit mehr als 30 Jahren eingesetzt. Der Faktor VIII:C/vWF-Komplex besteht aus zwei Molekülen, dem Antihämo-philie-Faktor (Faktor VIII:C) und dem von Willebrand-Faktor (vWF) . Beide Proteine werden unter der Kontrolle von verschiedenen Genen synthetisiert, zirkulieren aber im Plasma als nicht-kovalent gebundener Komplex (Faktor VIII : C/vWF-Komplex) .

Der Antihämophilie-Faktor ist ein Glykoprotein, welches in Hämophilie A-Patienten defekt ist, bzw. fehlt. Der von Willebrand-Faktor (vWF) ist ein multimeres Glykoprotein, welches in an von Willebrand-Krankheit erkrankten Patienten in reduzierter Menge oder qualitativ abnorm vorliegt. Die Plasmakonzentration von Faktor VIII:C beträgt etwa zwischen 100 und 200 ng/ml, während die Konzentration von vWF ungefähr 10 jug/ml beträgt.

Die Herstellung eines pharmazeutisch gut verträglichen FVIII:C/vWF-Komplexes sollte sich zum Ziele setzen, ein stabiles, vor allem aber auch ein von unerwünschten Begleitproteinen befreites Produkt zu liefern. Jede unnötige Proteinfracht birgt das Risiko von unerwünschten Nebenwirkungen in sich.

Im Stand der Technik sind verschiedene chromatographische Verfahren zur Gewinnung von Faktor VIII:C/vWF-Komplex beschrieben, beispielsweise mittels Anionenaustausch, Hydrophober- oder AffinitätsChromatographie.

Ein Verfahren zur Gewinnung des Faktor VIII:C/vWF-Komplexes aus humanem Plasma unter Verwendung einer Kationenaustausch-Chromatographie ist aus der EP-0 600 480 bekannt. Dabei werden mehrere Schritte zur Vorreinigung vorgenommen, darunter zwei chromatographische Reinigungen an einem Anionenaustauscher, die Kationenaustausch-Chromatographie wird terminal durchgeführt. Bei der mehrstufigen Reinigung werden erhebliche Aktivitätsverluste in Kauf genommen.

In der US-5 278 289 ist ein Verfahren zur Gewinnung von gereinigtem und stabilisiertem Faktor VIII aus einer biologischen Probe beschrieben. Dabei wurde die biologische Probe auf eine Kationenaustauscher-Säule aufgetragen, wobei aber nur ein unvollständig gereinigtes Protein erhalten wurde. Die vollständige Reinigung wird erst durch die nachgeschaltete Anionenaustauscher-Säule erzielt. Das Produkt enthält FVIII:C, der durch Dissoziation des Faktor VIII:C/vWF-Komplexes erhalten wurde.

Aufgabe der vorliegenden Erfindung ist es, den Faktor VIII:C/vWF-Komplex bzw. eine Faktor VIII:C/vWF-Komplex-Präparation durch ein einfaches chromatographisches Verfahren in hoher Reinheit und gleichzeitig mit hoher Ausbeute zur Verfügung .zu stellen.

Die Aufgabe wird erfindungsgemäß gelöst durch das eingangs angeführte Herstellungsverfahren mit den folgenden Schritten:
- Behandeln eines Faktor VIII:C/vWF-Komplex enthaltenden Ausgangsmaterials ausgewählt aus Plasma, Kryopräzipitat oder einer analogen Plasmafraktion mit einem Kationenaustauscher, wobei der Faktor VIII:C/vWF-Komplex an den Kationenaustauscher gebunden wird
- Abtrennen des nicht gebundenen Ausgangsmaterials und
- Eluieren des Faktor VIII:C/vWF-Komplexes mit einer mindestens 300fachen Reinheit bezogen auf Plasma und einer Ausbeute von mindestens 50% Aktivität des Faktor VIII:C und vWF gegenüber Kryopräzipitat oder analogen Plasmafraktionen erhalten wird, wobei die Chromatographie an einem Kationenaustauscher als einzige chromatographische Reinigung vorgenommen wird.

Als Kationenaustauschermaterialien können bevorzugterweise handelsübliche Kationenaustauschermaterialien mit Carboxy- oder Sulfhydrylgruppen verwendet werden. Vorzugsweise können S- oder DM-Sepharose (Fa. Amersham Pharmacia), Fractogel EMD-SO₃ ~ oder COO- (Fa. Merck, Darmstadt) oder SP- oder CM-Toyopearl (Fa. Tosohaas) verwendet werden.

Als Ausgangsmaterial kann Plasma oder eine Plasmafraktion verwendet werden. Als Plasmafraktion kann beispielsweise ein Kryopräzipitat, eventuell nach vorheriger Adsorptionsbehandlung zur Entfernung von Prothrombinkomplex-Faktoren, oder eine analoge Plasmafraktion, z.B. eine Cohn-Fraktion, wie Cohn I, oder eine entsprechende Fraktion nach Pool et al. (New England Journal of Medicine 273, 1443-1447) eingesetzt werden. Gegebenenfalls kann Fibrinogen durch Fällung entfernt sein.

Bevorzugterweise wird das Ausgangsmaterial in einem Puffer gelöst, der Kalziumionen enthält. Der Puffer kann auch Detergens enthalten. Die lonenstärke des Auftragepuffers beträgt im allgemeinen 0 - 10 mS, bevorzugt 6 - 8 mS. Falls der adsorbierte FVIII:C/vWF-Komplex gewaschen wird, wird diese Waschung bevorzugterweise mit einem Waschpuffer durchgeführt, dessen lonenstärke über der des Adsorptionspuffers liegt. Die Elution des Faktor VIII:C/vWF-Komplexes, der vorzugsweise nicht fraktioniert ist, wird durch diese Erhöhung der Ionenstärke erreicht. Die lonenstärke des Elutionspuffers beträgt üblicherweise 10 - 100 mS, bevorzugt 25 - 60 mS. Als Elutionspuffer können z.B. Acetatpuffer, Zitratpuffer oder Histidin-haltige Puffer eingesetzt werden. Vorzugsweise wird ein Natriumchlorid-haltiger Elutionspuf f er verwendet. Der pH sollte während der Reinigung etwa neutral, z.B. in einem Bereich von pH 6 - 8 gehalten werden.

Um eine etwaige Dissoziation des Faktor VIII:C/vWF-Komplexes während der chromatographischen Reinigung zu verhindern, wird bevorzugt ein Puffer mit einer Kalziumionenkonzentration von z.B. 0, 5 - 10 mM verwendet.

Zur Vermeidung des Risikos der Übertragung von humanpathogenen Infektionserregern, darunter die von Blut übertragbaren Viren, wie HIV und Hepatitisviren, z.B. HAV, HBV, HCV, HGV und Parvoviren, aber auch die infektiösen Erreger von BSE und CJD, kann eine Reihe von Maßnahmen vorgenommen werden. Der Faktor VIII:C/vWF-Komplex kann vor oder nach der chromatographischen Reinigung einem Verfahren zur Inaktivierung bzw. Abreicherung der Humanpathogene unterzogen werden. Die Methoden unter Einsatz von viruziden chemischen Substanzen werden vorzugsweise vor bzw. während des chromatographischen Reinigungsverfahrens vorgenommen, damit gleichzeitig mit der Reinigung des Faktor VIII:C/vWF-Komplexes auch das viruzide Mittel effektiv entfernt werden kann. Vorzugsweise werden mindestens zwei Maßnahmen vorgenommen, die die Inaktivierung bzw. Abreicherung der Viren aufgrund eines unterschiedlichen Mechanismus bewirken. Dazu zählen chemische, chemisch-physikalische und physikalische Methoden.

Zu den effektiven Mäßnahmen zur Inaktivierung von Viren zählen beispielsweise die Behandlung mit organischen Lösungsmitteln und/oder Detergentien (EP-0 131 740, EP-0 050 061), die Behandlung mit chaotropen Mitteln (EP-0 431 129), Hitzebehandlungsverfahren, vorzugsweise in lyophilisiertem, trockenem oder feuchtem Zustand, wie in der EP-0 159 311 beschrieben, Kombinationsverfahren, wie das der EP-0 519 901 und physikalische Methoden. Letztere bewirken die Inaktivierung von Viren beispielsweise mit Licht, etwa in Gegenwart von Photosensibilisatoren (EP-0 471 794 oder WO 97/37686) .

Zu den Abreicherungsverfahren der Humanpathogene zählen insbesondere auch die Filtration unter Verwendung von Ultrafiltern, Tiefenfiltern oder Nanofiltern (A 341/98). Aber auch Fällungsschritte bzw. andere Proteinreinigungsmaßnahmen, wie die der Adsorption, tragen zur Abreicherung von möglicherweise vorhandenen Pathogenen bei.

Das erfindungsgemäße Verfahren eignet sich hervorragend zur Reinigung des Faktor VIII:C/vWF-Komplexes aus einer Plasmafraktion im industriellen Maßstab, da aufgrund der effektiven einstufigen Reinigung eine Vielzahl von weiteren Reinigungsschritten, wie z.B. weitere chromatographische Reinigungsschritte, nicht erforderlich sind. Gerade bei der großtechnischen Herstellung von biologischen Präparaten ist ein einfaches, einstufiges Verfahren, wie das erfindungsgemäße Verfahren, bei welchem derart hohe Ausbeuten und eine derart hohe Reinheit erzielt werden können, besonders erwünscht.

Es hat sich überraschenderweise gezeigt, daß der Faktor VIII:C/vWF-Komplex durch die einfache Kationenaustausch-Chromatographie mit einer mindestens 300-fachen Reinheit gegenüber Plasma, vorzugsweise mindestens 400-fachen Reinheit, erhalten werden kann, mit einer gleichzeitig hohen Ausbeute an FVIII:C und vWF von mindestens 50%, vorzugsweise mindestens 60%, am meisten bevorzugt mindestens 70%, bezogen auf Kryopräzipitat oder analoge Plasmafraktionen.

Es hat sich gezeigt, daß die Ausbeute des Faktor VIII:C/vWF-Komplexes von mehr als 90% bezogen auf die Aktivität vor der Chromatographie erhalten werden kann. Daher kann sogar während der chromatographischen Reinigung auf übliche Stabilisatoren des Faktor VIII:C/vWF-Komplexes, wie z.B. Antithrombin III und/oder Heparin verzichtet werden, ohne beträchtliche Verluste durch Denaturierung befürchten zu müssen.

Die Aktivität des Faktor VIII:C bzw. vWF wird durch die Kätionenaustausch-Chromatographie kaum beeinträchtigt. Faktor VIII:C-Aktivität kann z.B. mittels chromogenem Assay (Immunochrom FVIII:C, Fa. IMMUNO AG) bestimmt werden. Die Aktivität des vWF kann durch einen Collagenbindungs-Test nach Thomas et al. (Haemostaseologie, 14, 133-139 (1994)) oder mittels ELISA bestimmt werden.

Zur Formulierung einer pharmazeutischen Faktor VIII:C/vWF-Komplex-Präparation wird üblicherweise diafiltriert und sterilfiltriert, sowie gegebenenfalls lyophilisiert.

Die Erfindung wird anhand des nachfolgenden Beispiels noch näher erläutert.

### Beispiel 1:

### Isolierung des FVIII/vWF-Komplexes über Kationenaustausch

### Beispiel 1A:

210 g Kryopräzipitat werden in 950 ml CaCl₂-heparinhaltigem Citratpuffer gelöst und auf pH 6, 0 gestellt. Unlösliche Anteile, hauptsächlich Fibrinogen, wurden abtrennt. Zur Inaktivierung von eventuell vorhandenen pathogenen Viren wurde die klare Lösung mit 1 % Triton X100 und 0, 3 % TNBP (Tri (n-butyl) phosphat behandelt. 100 ml Fractogel EMD-SO₃⁻-650 (M) der Fa. Merck, Darmstadt (DE), wurden zur Adsorption des virusinaktivierten FVIII herangezogen, das zuvor bei pH 6,0 in einer acetatgepufferten NaCl-Lösung mit einer Leitfähigkeit von 10 mS/cm äquilibriert wurde. Der FVIII wurde durch Erhöhung der Ionenstärke auf 500 mM NaCl vom Gel abeluiert; vorher wurde eine Waschung mit 500 ml 150 mM NaCl-Lösung durchgeführt.

### Beispiel 1B:

Statt Fractogel EMD-SO₃⁻ wurde in diesem Beispiel Toyopearl SP-550C verwendet.

### Ergebnisse:

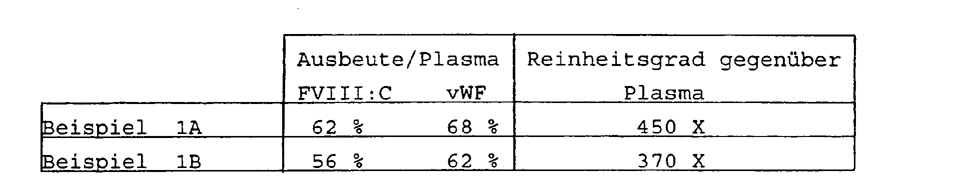

FVIII:C und vWF sind in derselben Fraktion gewonnen worden.

## Patentansprüche

1. Verfahren zur Herstellung einer Präparation enthaltend Faktor VIII:C/vWF-Komplex **gekennzeichnet durch** die folgenden Schritte:
- Behandeln eines Faktor VIII:C/vWF-Komplex enthaltenden Ausgangsmaterials ausgewählt aus Plasma, Kryopräzipitat oder einer analogen Plasmafraktion mit einem Kationenaustauscher, wobei der Faktor VIII:C/vWF-Komplex an den Kationenaustauscher gebunden wird
- Abtrennen des nicht gebundenen Ausgangsmaterials und
- Eluieren des Faktor VIII:C/vWF-Komplexes mit einer mindestens 300fachen Reinheit bezogen auf Plasma und einer Ausbeute von mindestens 50% Aktivität des Faktor VIII:C und vWF gegenüber Kryopräzipitat oder analogen Plasmafraktionen erhalten wird, wobei die Chromatographie an einem Kationenaustauscher als einzige chromatographische Reinigung vorgenommen wird.

2. Verfahren nach Anspruch 1, **dadurch gekennzeichnet, dass** die Chromatographie in Gegenwart von viruziden Substanzen vorgenommen wird.

3. Verfahren nach Anspruch 1 oder 2, **dadurch gekennzeichnet, dass** der Faktor VIII:C/vWF-Komplex einstufig eluiert wird.

## Claims

1. A method of producing a preparation containing factor VIII:C/vWF-complex, **characterized by** the following steps:
- treating a factor VIII:C/vWF-complex containing starting material selected from plasma, cryoprecipitate or an analogous plasma fraction with a cation exchanger, whereby the factor VIII:C/vWF-complex is bound to the cation exchanger,
- separating the non-bound starting material, and
- eluting the factor VIII:C/vWF-complex with an at least 300-fold purity, based on plasma, and a yield of at least 50% activity of factor VIII:C and vWF relative to cryoprecipitate or analogous plasma fractions, wherein the chromatography is carried out on a cation exchanger as the sole chromatographic purification.

2. A method according to claim 1, **characterised in that** the chromatography is carried out in the presence of virucidal substances.

3. A method according to claim 1 or 2, **characterised in that** the factor VIII:C/vWF-complex is eluted in one step.

## Revendications

1. Procédé de production d'une préparation contenant un complexe facteur VIII:C/vWF, **caractérisé par** les étapes suivantes :
- traitement d'un matériau de départ contenant un complexe facteur VIII:C/vWF choisi parmi un plasma, un cryoprécipité ou une fraction plasmatique analogue, avec un échangeur de cation, le complexe facteur VIII:C/vWF étant lié à l'échangeur de cation,
- séparation du matériau de départ non lié et
- élution du complexe facteur VIII:C/vWF avec une pureté d'au moins un facteur 300 par rapport au plasma et un rendement d'au moins 50 % de l'activité du complexe facteur VIII:C/vWF à l'encontre du cryoprécipité ou de fractions plasmatiques analogues étant obtenu, la chromatographie étant réalisée sur un échangeur de cation comme purification chromatographique unique.

2. Procédé selon la revendication 1, **caractérisé en ce que** la chromatographie est réalisée en présence de substances virucides.

3. Procédé selon la revendication 1 ou 2, **caractérisé en ce que** le complexe facteur VIII:C/vWF est élué en une étape.
